# EUROPÄISCHE PATENTSCHRIFT

(11) **EP 0 951 236 B1**
(45) Veröffentlichungstag und Bekanntmachung des Hinweises auf die Patenterteilung: **02.04.2003**
(21) Anmeldenummer: 97951946.9
(22) Anmeldetag: 21.11.1997
(51) Int. Cl.: A61B 10/00

(54) **EPIKUTAN-TESTPFLASTER**
EPICUTANEOUS TEST PLASTER
PANSEMENT POUR TEST EPICUTANE

(30) Priorität: 11.12.1996 DE 29621365 U
(43) Veröffentlichungstag der Anmeldung: 27.10.1999
(73) Patentinhaber: Lohmann GmbH & Co. KG, 56567 Neuwied (DE)
(72) Erfinder: ANHÄUSER, Dieter, D-56581 Melsbach (DE); ECKER, Jürgen, D-56567 Neuwied (DE); SCHENTEK, Heike, D-56581 Kurtscheid (DE)
(74) Vertreter: Flaccus, Rolf-Dieter, Dr.
(86) Internationale Anmeldenummer: EP9706532
(87) Internationale Veröffentlichungsnummer: WO98025521

(56) Entgegenhaltungen:
- WO-A-94/17735
- DE-C- 3 810 658
- DE-C- 4 328 112

## Beschreibung

Die Erfindung betrifft eine Applikationshilfe für Epikutantestpflaster mit mindestens einer auf einer Trägerfläche angeordneten Wirkstoffaufnahmeeinrichtung.

Epikutantestpflaster werden bei Epikutantests eingesetzt, die besonders zur Aufklärung der Ursachen allergischer Kontaktekzeme dienen. Zu diesem Zweck wird die zu prüfende Testsubstanz auf ein Testpflaster aufgetragen, das dann auf ausgesuchte Hautstellen des Patienten appliziert wird. Nach einem vorherbestimmten Zeitraum wird das Testpflaster abgezogen.

Die erste Ablesung der Reaktion des Patienten auf die Wirkstoffexposition erfolgt unmittelbar nach Abnahme des Testpflasters; gegebenenfalls erfolgen weitere Ablesungen in bestimmten Zeitabständen. Dabei ist eine Markierungsmöglichkeit der Teststelle auf der Haut hilfreich, um die mit der Testsubstanz kontaktierte Hautstelle zuverlässig wiederfinden zu können.

Der prinzipielle Aufbau von Epikutantestpflastern ist folgender: auf einer haftklebenden Trägerschicht sind als Wirkstoffaufnahmeeinrichtungen absorptionsfähige Flächengebilde, beispielsweise aus Geweben oder nicht gewebten Stoffen, oder zur Haut hin offene Behältnisse angeordnet oder in der Trägersubstanz selbst ausgebildet.

Die mit der Haut in Kontakt zu bringende Fläche des Testpflasters ist vor der Applikation durch eine ablösbare Schutzschicht abgedeckt. Als Trägerschicht wurden bisher textile Flächenmaterialien, wie beispielsweise Gewebe oder Vliesstoffe, sowie Polymere oder Metallfolien vorgeschlagen.

Ein wesentlicher Nachteil bekannter Epikutantestpflaster bestand darin, daß die Trägermaterialien eine zu geringe Flexibilität besaßen, um das vorzeitige Ablösen des Testpflasters von der Haut durch unvermeidbare Körperbewegungen zu unterbinden. Zur Behebung dieses Nachteils wird in der DE-PS 38 10 658 ein Trägermaterial aus einer dünnen, für flüssiges Wasser dichten, aber für Wasserdampf durchlässigen, hochelastischen Polymerfolie vorgeschlagen, deren Handhabbarkeit durch Anbringen einer wieder ablösbaren steiferen Stützfolie auf der hautabgewandten Fläche der Trägerfolie sichergestellt wird. Die Stützfolie wird nach der Applikation abgezogen.

Trotz dieses Fortschrittes kann aber die Handhabbarkeit derartiger Testpflaster bisher nicht ganz befriedigen. Bei der Applikation muß eine Anfaßmöglichkeit des Pflasters gegeben sein, bei der die Kontamination der Haftklebeschicht, natürlich inklusive der Wirkstoffaufnahmevorrichtungen, vermieden werden. Ebenso muß die Stützfolie nach Aufkleben des Pflasters bequem abziehbar sein. Eine die Trägerschicht überragende Stützfolie kann hier zwar Abhilfe bringen, erfordert aber einen erhöhten Materialbedarf und zusätzliche und/oder aufwendige Arbeitsschritte bei der Herstellung.

Es ist daher Aufgabe der Erfindung, ein Epikutantestplaster zu schaffen, das sich bei guter Anschmiegsamkeit durch eine mit vertretbaren Mitteln erreichbare akzeptable Handhabbarkeit auszeichnet.

Überraschenderweise wurde die Lösung dieser Aufgabe darin gefunden, daß bei einem Epikutanpflaster mit mindestens einer auf einer Trägerschicht angeordneten Wirkstoffaufnahmeeinrichtung mit einer für flüssiges Wasser dichten, aber für Wasserdampf durchlässigen, hochelastischen Trägerfolie aus Polymermaterial, welche an der der Haut abgewandten Fläche mit einer Stützfolie lösbar verbunden und auf der der Haut zugewandten Fläche mit einer Haftklebeschicht versehen ist, die ihrerseits vor der Applikation des Pflasters mit einer mindestens zweiteiligen ablösbaren Schutzschicht abgedeckt ist, die Schutzschicht an zwei gegenüberliegenden Rändern scharnierartig mit einer Stützfolie verbunden ist.

Diese Konstruktion erlaubt es unter anderem, die Stützfolie mit der Trägerfolie flächengleich zu gestalten und somit zusätzliche Schneid- und/oder Stanzvorgänge zu vermeiden. Die Schutzschichtteile lassen sich nach Ablösen von der Haftklebeschicht nach außen klappen und können zur Applikation des Pflasters angefaßt werden, ohne daß eine Kontamination der Haftklebeschicht in Kauf genommen werden muß. Nach Andrücken des Pflasters an die Haut kann die Stützfolie mit den anhängenden Teilen der Schutzschicht problemlos abgezogen werden.

Ein weiterer Vorteil der Erfindung liegt darin, daß nach Aufklappen der Schutzschichtteile die Wirkstoffaufnahmeeinrichtungen mit den zu prüfenden Substanzen befüllt werden können und bis zum Zeitpunkt der Applikation durch Rückklappen der Schutzschichtteile in die Ausgangsposition gegen Einwirkungen von außen geschützt sind.

Für die Realisierung der scharnierartigen Verbindung zwischen Schutzschicht und Stützfolie gibt es zahlreiche Möglichkeiten, von denen einige besonders bevorzugte beschrieben werden. Einmal kann die Schutzschicht streifenförmig den Rand des Pflasters überragen und ist nach Umknicken um diesen Rand auf der hautabgewandten Fläche der Stützfolie adhäsiv befestigt. Eine Faltlinie in der Schutzschicht begünstigt deren Beweglichkeit um die Scharnierachse. Die umgekehrte Konstruktion, d. h. daß die überstehende Stützfolie nach Umknicken um den Rand auf der Unterseite der Schutzschicht befestigt ist, ist prinzipiell auch möglich, wird aber wegen eventueller Nachteile auf Sonderfälle beschränkt bleiben.

Im dem Falle, wo es die Umstände erlauben, die Stützfolie und die Schutzschicht aus demselben Material herzustellen, wird ein zusammenhängendes Flächengebilde um die Trägerfläche herum gefaltet, so daß die Enden auf der Seite der Klebeschicht zu liegen kommen. Die Adhäsion der Trägerfolie an diesem Flächengebilde wird nach bekannten, weiter unter näher beschriebenen Methoden geschaffen.

Bei einer weiteren bevorzugten Ausgestaltung der Erfindung haben Stützfolie, haftkleberbeschichtete Trägerfolie und Schutzschicht dieselben Außenkonturen und die scharnierartige Verbindung zwischen Schutzschicht und Stützfolie wird durch Anbringen eines längs geknickten Streifens gebildet, wobei ein Teil auf der hautabgewandten Seite der Stützfolie und der andere Teil auf der freien Seite der Schutzschicht haftet. Der Streifen ist vorzugsweise haftklebend ausgerüstet, kann aber auch durch andere Klebersysteme fixiert werden. Das Auf siegeln bei Zimmertemperatur oder unter Wärmezufuhr bietet weitere Möglichkeiten der Fixierung. Selbstverständlich braucht der Streifen nicht die gesamte Länge der Scharnierkante zu umschließen, sondern es genügt in manchen Fällen, wenn nur Teile der Kante belegt sind.

Vorzugsweise weist das Epikutantestpflaster gemäß der Erfindung eine viereckige Kontur auf, wobei an gegenüberliegenden Kanten -bevorzugt sind die Längskanten- die scharnierartigen Verbindungen gebildet sind. Die Größe der Trägerfolie entspricht der Größe der Stützfolie nicht, wenn parallel zu mindestens einem Rand des Pflasters ein hautseitig haftklebender, mit hautabgewandter Fläche an der über die Trägerfolie hinausragenden Stützfolie lösbar befestigter Markierungsstreifen angeordnet ist. Dieser Steifen klebt nach der Applikation des Pflasters neben diesem auf der Haut und eröffnet die Möglichkeit, teststellenbezogene Markierungen vorzunehmen.

Als Material für die Stützfolie kommen bekannte Polymere in Frage, wie z. B. Polyethylen, Polypropylen, Polyamid, oder Polyester. Daneben sind aber auch textile Flächengebilde und Papier in Gebrauch. Die Schichtdicke der Stützfolie bewegt sich zwischen 30 und 200 µm , vorzugsweise 30 -80 µm. Die Stützfolie ist mit der Trägerfolie mit einem geeigneten Kleber verklebt oder die Verbindung wird durch jene mechanischen Adhäsionskräfte bewerkstelligt, die entstehen, wenn die Trägerfolie durch Extrusion, Gießen oder eine andere bekannte Art der Folienherstellung unmittelbar auf der Stützfolie erzeugt wird.

Die Trägerfolie ist opak oder transparent und kann nach bekannten Techniken aus bekannten Rohstoffen hergestellt werden. Transparente Folien sind bevorzugt, da sie die Beobachtung der Testpflaster während der Applikation erlauben. Die besten Resultate in dieser Hinsicht werden erhalten, wenn zusätzlich auch die Wirkstoffaufnahmeeinrichtungen transparent gestaltet sind. Als Rohstoffe für die Trägerfolie seien beispielsweise angeführt: Polyurethane, Polyvinylchloride, Polyvinylidenchloride, Polyvinylalkohole, Polyacrylate, Polysulfone, Polystyrole, Poyethylen,Polypropylen, Polyamide, Ethylen-Vinylacetat-Copolymere, Polyester, Polycarbonate, PoLyvinylfluorid und andere fluorhaltige Polymere. Besonders bevorzugt sind Trägerfolien auf Basis von Polyurethan. Allgemein liegt die Schichtdecke geeigneter Folien im Bereich von 7 - 120 µm, bevorzugt von 15 - 50 µm. Die Wasserdampfdurchlässigkeit soll bei mindestens 300 g x m⁻² x 24h⁻¹ liegen.

Die Haftklebeschicht kann aus bekannten physiologisch unbedenklichen Materialien bestehen. Als Beispiele genannt seien Kautschuk, kautschukähnliche synthetische Homo-, Cooder Blockpolymere, Polyacrylate und entsprechende Copolymerisate, Polyurethane und Silikone. Der Flächenauftrag des Haftklebers liegt zwischen 15 - 80 g/m², vorzugsweise 30-50 g/m².

Das Material für die Schutzschicht kann jenes sein, das auch für die Stützfolie Verwendung findet. Darüberhinaus können aber auch beispielsweise Polytetrafluorethylen, Cellophan, Polyvinylchlorid, dehäsiv behandelte Papiere, Metallfolien und mit Polymeren überzogene Metallfolien eingesetzt werden. Die Flächengewichte liegen bei 30 - 250 g/m², vorzugsweise bei 50 - 150 g/m². Die mit der Haftklebeschicht in Kontakt befindliche Seite der Schutzschicht muß eine Wiederablösung mit einer Kraft erlauben, die kleiner ist als jene zur Ablösung der Stützfolie von der Trägerfolie. Die Enden der mindestens zweiteiligen Schutzschicht, die auf die Haftklebeschicht zu liegen kommen, sind tunlichst in gewohnter Weise mit Anfaßhilfen ausgestattet.

Die Wirkstoffaufnahmeeinrichtungen sind rund, eckig, oval, oder in einer anderen Flächenform ausgelegt und bestehen in der Regel aus absorptionsfähigem Material, wie beispielsweise Papier, Vliesstoff, Gewebe oder gelartigen Polymeren, die den Wirkstoff an die Haut abgeben können. Daneben finden auch schälchenartige Vorrichtungen zur Aufnahme der Wirkstoffe Verwendung. Zur transparenten Ausgestaltung der Aufnahmeeinrichtungen hat sich die Verwendung von Cellophan oder Polyvinylalkoholpolymeren bewährt. Im allgemeinen werden die Wirkstoffaufnahmeeinrichtungen in an sich bekannter Weise auf der Haftklebeschicht befestigt. Um eine unbeabsichtigte Wanderung von flüssigen Testsubstanzen aus der Aufnahmeeinrichtung zu verhindern, kann eine Folienumrandung vorgesehen werden, die etwa mit halber Breite die Aufnahmeeinrichtung abdeckt und mit der anderen Hälfte ihrer Breite auf der Haftklebeschicht befestigt ist, ohne daß die vorgesehene Hautkontaktfläche der Wirkstoffaufnahmeeinrichtung beeinträchtigt wird.

Die Epikutantestpflaster gemäß der Erfindung können als Einzelpflaster, d. h. mit nur einer Wirkstoffaufnahmeeinrichtung, ausgebildet sein, weisen aber vorzugsweise mehrere nach einem vorbestimmten geometrischen Muster, bevorzugt reihenförmig, angeordnete Wirkstoffaufnahmeeinrichtungen auf. Die Reihe bzw. gewünschtenfalls zwei oder mehrere parallel verlaufende Reihen erstrecken sich in Längsrichtung des Pflasters. Zwischen den einzelnen Aufnahmeeinrichtungen können Sollbruchlinien vorgesehen werden, die ein leichtes Abtrennen eines Pflasters mit der gwünschten Anzahl von Aufnahmeeinrichtungen von einer längeren Pflasterbahn ermöglichen.

Die Erfindung wird anhand von Figuren weiter erläutert. Sie sind nicht maßstabsgetreu dargestellt, und die Dicke der vorkommenden Schichten ist zum besseren Verständnis übertrieben stark wiedergegeben. Gleiche Elemente tragen die gleichen Bezugszeichen.

Es zeigen:
- Fig. 1: eine Draufsicht auf ein erfindungsgemäßes Epikutantestpflaster
- Fig. 2 und 3: einen Querschnitt gemäß der Linie II/II in Fig. 1 und
- Fig. 3 und 4: je ein Querschnittsfragment mit verschiedenen Scharnierausführungen

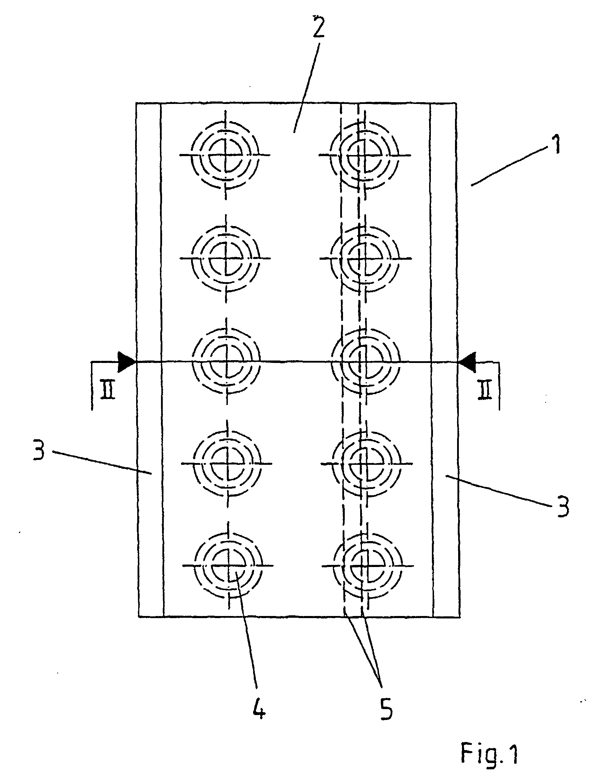

Das Epikutantestpflaster in Fig. 1 ist mit 1 bezeichnet. Es ist in der Draufsicht dargestellt, so daß von den das Pflaster bildenden Schichten nur die Stützfolie 2 zu sehen ist. Sie wird an gegenüberliegenden Rändern von je einem Streifen 3 abgedeckt, der die scharnierartige Verbindung mit der Schutzschicht bildet. Die unterbrochenen Kreise 4 geben die Stellen an, wo auf der Unterseite des Pflasters die Wirkstoffaufnahmevorrichungen angebracht sind. Im vorliegenden Falle sind je fünf Vorrichtungen in zwei parallelen Reihen angeordnet. Die gestrichelten Linien 5 markieren den Verlauf der mit der Anfaßhilfe ausgestalteten Schutzschichtteile auf der entgegengesetzten Seite des Pflasters.

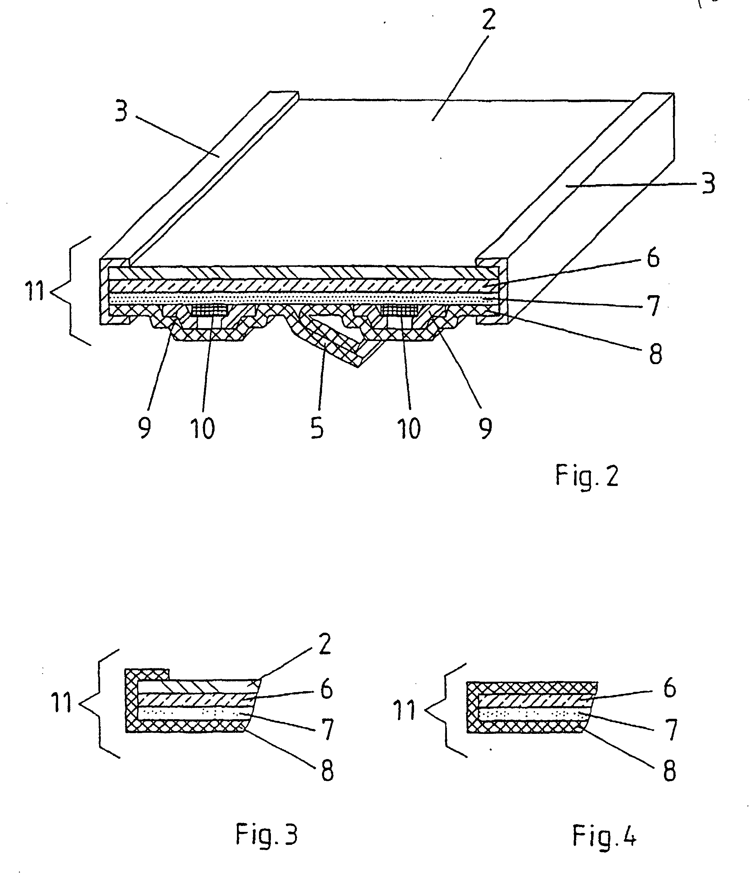

Fig. 2 zeigt in perspektivischer Querchnittsdarstellung nach Linien II/II in Fig. 1 ein erfindungsgemäßes Epikutantestpflaster. Die hochelastische Trägerfolie 6 ist auf der hautabgewandten Seite von der gleich großen Stützfolie 2 abgedeckt. Auf der hautzugewandten Seite der Trägerfolie 6 ist eine Haftklebaschicht 7 angeordnet, auf der die Wirkstoffaufnahmevorrichtungen aus einem Ring 9 mit Absorptionsmaterial 10 befestigt sind. Die Schutzschicht ist mit 8 bezeichnet. Wobei die Anfaßhilfe 5 für deren beide Teile zu erkennen sind.

Weitere Möglichkeiten der Scharnierbildung 11 sind in den Fig. 3 und 4 wiedergegeben. An den Fragmenten von Querschnitten ist ersichtlich, daß in Fig. 3 die Schutzschicht 8'selbst das Scharniermaterial bildet und nach Umschlingung der Kante auf der Oberseite der Stützfolie 2 befestigt ist. In Fig. 4 sind Schutzschicht 8 und Stützfolie aus einem einheitlichen Material und erlauben es daher, durch einfache Faltung über die Kanten die gewünschte scharnierartige Verbindung 11 zwischen den Außenschichten des Pflasters herzustellen.

## Patentansprüche

1. Epikutantestpflaster (1) mit mindestens einer auf einer Trägerfolie angeordneten Wirkstoffaufnahmeeinrichtung (4), mit einer für flüssiges Wasser dichten, für Wasserdampf durchlässigen, hochelastischen Trägerfolie (6) aus Polymermaterial, die an der der Haut abgewandten Fläche mit einer Stützfolie (2)lösbar verbunden und auf der der Haut zugewandten Fläche mit einer Haftklebeschicht (7) versehen ist, die ihrerseits vor der Applikation des Pflasters mit einer mindestens zweiteiligen ablösbaren Schutzschicht (8) abgedeckt ist, **dadurch gekennzeichnet, daß** die Schutzschicht (8) an zwei gegenüberliegenden Rändern scharnierartig (11) mit der Stützfolie (2) verbunden ist.

2. Epikutantestpflaster nach Anspruch 1, **dadurch gekennzeichnet, daß** die scharnierartige Verbindung (11) zwischen Schutzschicht (8) und Stützfolie (2) durch adhäsives Befestigen eines streifenförmigen, den Rand des Pflasters überragenden Teiles der Schutzschicht entlang einer Faltlinie auf der hautabgewandten Fläche der Stützfolie (2) gebildet ist.

3. Epikutantestpflaster nach Anspruch 1, **dadurch gekennzeichnet, daß** die Schutzschicht (8) und die Stützfolie (2) aus demselben Material bestehen und zusammenhängend über zwei gegenüberliegenden Rändern des Pflasters unter Ausbildung eines Scharniers (11) gefaltet sind, wobei die Enden des Flächengebildes auf die Seite der Schutzschicht (8) zu liegen kommen.

4. Epikutantestpflaster nach Anspruch 1, **dadurch gekennzeichnet, daß** die scharnierartige Verbindung (11) zwischen Schutzschicht (8) und Stützfolie (2) durch einen aufgesetzten, längs geknickten Streifen (3) gebildet ist.

5. Epikutantestpflaster nach Anspruch 4, **dadurch gekennzeichnet, daß** der Streifen (3) aufgeklebt ist.

6. Epikutantestpflaster nach Anspruch 4, **dadurch gekennzeichnet, daß** der Streifen (3) aufgesiegelt ist.

7. Epikutantestpflaster nach Ansprüchen 4 bis 6, **dadurch gekennzeichnet, daß** der Streifen (3) über die Länge des Pflasterrandes in voneinander getrennte Abschnitte unterteilt ist.

8. Epikutantestpflaster nach einem oder mehreren der vorangehenden Ansprüche, **dadurch gekennzeichnet, daß** es in Längsrichtung eine Reihe von Wirkstoffaufnahmeeinrichtungen (4) umfaßt.

9. Epikutantestpflaster nach Anspruch 8, **dadurch gekennzeichnet, daß** es in Längsrichtung mindestens zwei parallele Reihen von Wirkstoffaufnahmeeinrichtungen (4) umfaßt.

10. Epikutantestpflaster nach einem oder mehreren der vorangehenden Ansprüche, **dadurch gekennzeichnet, daß** die Trägerfolie (6) kleiner dimensioniert ist als die Stützfolie (2).

11. Epikutantestpflaster nach Anspruch 10, **dadurch gekennzeichnet, daß** parallel zu mindestens einem Rand des Pflasters ein hautseitg haftklebender, mit der hautabgewandten Fläche an der über die Trägerfolie hinausragenden Stützfolie lösbar befestigter Markierungsstreifen angeordnet ist.

## Claims

1. Patch test plaster (1) having at least one active substance receptacle (4) arranged on a backing film, having a highly elastic backing film (6) which is made of polymeric material, is impermeable to liquid water but permeable to water vapour, and is releasibly connected on the surface facing away from the skin to a supporting sheet (2) and is provided on the surface facing the skin with an adhesive layer (7) which is in turn covered by an at least two-part detachable protecting layer (8) before application of the plaster, **characterized in that** the protecting layer (8) has a hinge-like connection (11) on two opposite edges to the supporting sheet (2).

2. Patch test plaster according to Claim 1,
**characterized in that** the hinge-like connection (11) between protecting layer (8) and supporting sheet (2) is formed by adhesive fastening of a strip-like part, which projects beyond the edge of the plaster, of the protecting layer along a crease line on the surface of the supporting sheet (2) facing away from the skin.

3. Patch test plaster according to Claim 1,
**characterized in that** the protecting layer (8) and the supporting sheet (2) consist of the same material and are folded continuously over two opposite edges of the plaster to form a hinge (11), with the ends of the flat material coming to rest on the side of the protecting layer (8).

4. Patch test plaster according to Claim 1,
**characterized in that** the hinge-like connection (11) between protecting layer (8) and supporting sheet (2) is formed by an attached, longitudinally creased strip (3).

5. Patch test plaster according to Claim 4,
**characterized in that** the strip (3) is stuck on.

6. Patch test plaster according to Claim 4,
**characterized in that** the strip (3) is sealed on.

7. Patch test plaster according to Claims 4 to 6,
**characterized in that** the strip (3) is divided into mutually separate sections over the length of the edge of the plaster.

8. Patch test plaster according to one or more of the preceding claims, **characterized in that** it comprises in the axial direction a row of active substance receptacles (4).

9. Patch test plaster according to Claim 8,
**characterized in that** it comprises in the axial direction at least two parallel rows of active substance receptacles (4).

10. Patch test plaster according to one or more of the preceding claims, **characterized in that** the backing film (6) has smaller dimensions than the supporting sheet (2).

11. Patch test plaster according to Claim 10,
**characterized in that** a marking strip which is adhesive on the skin side and is releasibly fastened on the surface facing away from the skin to the supporting sheet which projects beyond the backing film is arranged parallel to at least one edge of the plaster.

## Revendications

1. Pansement pour test épicutané (1) avec au moins un dispositif d'absorption de substance active (4) disposé sur une feuille porteuse, avec une feuille porteuse (6) hautement élastique en polymère, étanche à l'eau liquide et perméable à la vapeur d'eau, cette feuille porteuse étant raccordée à une feuille de maintien (2) de manière détachable sur la surface détournée de la peau et étant munie d'une couche auto-adhésive (7) sur la surface tournée vers la peau, qui, de son côté, est recouverte, avant l'application du pansement, d'une couche protectrice (8) détachable au moins en deux parties, **caractérisé en ce que** la couche protectrice (8) est raccordée à la feuille de maintien (2) sur deux bords opposés, de manière similaire à une charnière (11).

2. Pansement pour test épicutané selon la revendication 1, **caractérisé en ce que** le raccordement similaire à une charnière (11) est formé entre la couche protectrice (8) et la feuille de maintien (2) par la fixation adhésive d'une partie de la couche protectrice en forme de bande et dépassant du bord du pansement, le long d'une ligne de pliage sur la surface détournée de la peau de la feuille de maintien (2).

3. Pansement pour test épicutané selon la revendication 1, **caractérisé en ce que** la couche protectrice (8) et la feuille de maintien (2) sont constituées de la même matière et sont pliées en tenant ensemble sur deux bords opposés du pansement en formant une charnière (11), les extrémités de la surface textile venant reposer sur le côté de la couche protectrice (8).

4. Pansement pour test épicutané selon la revendication 1, **caractérisé en ce que** le raccordement similaire à une charnière (11) entre la couche protectrice (8) et la feuille de maintien (2) est formé par une bande (3) pliée en longueur et posée par-dessus.

5. Pansement pour test épicutané selon la revendication 4, **caractérisé en ce que** la bande (3) est collée.

6. Pansement pour test épicutané selon la revendication 4, **caractérisé en ce que** la bande (3) est scellée.

7. Pansement pour test épicutané selon les revendications 4 à 6, **caractérisé en ce que** la bande (3) est divisée en sections séparées les unes des autres sur la longueur du bord du pansement.

8. Pansement pour test épicutané selon une ou plusieurs des revendications précédentes, **caractérisé en ce qu'**il comprend une série de dispositifs d'absorption de substance active (4) dans le sens longitudinal.

9. Pansement pour test épicutané selon la revendication 8, **caractérisé en ce qu'**il comprend au moins deux rangées parallèles de dispositifs d'absorption de substance active (4) dans le sens longitudinal.

10. Pansement pour test épicutané selon l'une ou plusieurs des revendications précédentes, **caractérisé en ce que** la feuille porteuse (6) est de dimension inférieure à la feuille de maintien (2).

11. Pansement pour test épicutané selon la revendication 10, **caractérisé en ce qu'**une bande de marquage auto-adhésive du côté de la peau, fixée de manière détachable avec la surface détournée de la peau sur la feuille de maintien dépassant de la feuille porteuse, est disposée en parallèle à au moins un bord du pansement.
